# EUROPÄISCHE PATENTSCHRIFT

(11) **EP 0 616 608 B1**
(45) Veröffentlichungstag und Bekanntmachung des Hinweises auf die Patenterteilung: **12.03.1997**
(21) Anmeldenummer: 92924646.0
(22) Anmeldetag: 02.12.1992
(51) Int. Cl.: C07C 67/48, C07C 69/54

(54) **VERBESSERTE TROCKENNEUTRALISATION OLEFINISCH REAKTIVER ORGANISCHER FLÜSSIGPHASEN**
IMPROVED METHOD FOR THE DRY-NEUTRALIZATION OF OLEFINICALLY REACTIVE ORGANIC LIQUIDS
NEUTRALISATION A SEC AMELIOREE DE PHASES LIQUIDES ORGANIQUES A REACTION OLEFINIQUE

(30) Priorität: 07.12.1991 DE 4140373
(43) Veröffentlichungstag der Anmeldung: 28.09.1994
(73) Patentinhaber: Henkel Kommanditgesellschaft auf Aktien, 40191 Düsseldorf (DE)
(72) Erfinder: RITTER, Wolfgang, D-5657 Haan (DE); ORTANDERL, Stefanie, D-4053 Jüchen 1 (DE)
(86) Internationale Anmeldenummer: EP9202785
(87) Internationale Veröffentlichungsnummer: WO9312066

(56) Entgegenhaltungen:
- WO-A-90/07484
- DE-A- 2 330 435

## Beschreibung

Die Erfindung betrifft eine weiterführende Verbesserung bei der Reingewinnung von im wesentlichen neutralen schwerflüchtigen und polymerisations- bzw. vergelungs-gefährdeten organischen Komponenten, die reaktive olefinische Doppelbindungen aufweisen. Die Erfindung will dabei insbesondere die Reingewinnung solcher Komponenten ermöglichen, deren Reinigung auf destillativem Wege nicht oder nicht ohne weiteres möglich ist.

Die der Erfindung zugrundeliegende Problematik wird im nachfolgenden beispielhaft an der Stoffklasse der Polyolacrylsäureester beschrieben. Für den Fachmann sofort einleuchtend gilt allerdings, daß der Umfang des Anwendungsgebietes der Erfindung sich nicht auf diese bestimmte Stoffklasse einschränkt. Ein weiteres Beispiel sind entsprechende Ester der Crotonsäure.

Zur Herstellung von Polyol(meth)acrylsäureestern werden mehrfunktionelle Alkohole mit Acrylsäure und/oder Methacrylsäure - im nachfolgenden als (Meth)acrylsäure bezeichnet - in Gegenwart geeigneter Katalysatoren und Inhibitoren und in Anwesenheit eines Schleppmittels, zum Beispiel Toluol und/oder Xylol, verestert. Nachteilig ist die Mitverwendung von Lösungs- bzw. Schleppmitteln und die Notwendigkeit, das Rohprodukt durch alkalische Wäsche von (Meth)acrylsäure-Überschüssen und dem sauren Katalysator - üblicherweise p-Toluolsulfonsäure - zu befreien.

In den DE-A1 38 43 854, 38 43 938, 38 43 930 und 38 43 843 werden verbesserte Möglichkeiten zur Herstellung solcher polyfunktioneller (Meth)acrylsäureester ohne Mitverwendung von Lösungsmitteln beschrieben. Neben dieser Möglichkeit, auf Lösungsmittel völlig zu verzichten, liegt ein wichtiger Verfahrensvorteil auch darin, im Reaktionsrohgemisch vorliegende saure Anteile - beispielsweise Restmengen der (Meth)acrylsäure, saure Katalysatoren und/oder sauer reagierende Inhibitoren - ohne Wäsche durch eine sogenannte Trokkenneutralisation zu entfernen. Einzelheiten zu dieser Verfahrensmodifikation sind in den DE-A1 39 39 162 und 39 39 163 beschrieben.

Das beispielsweise in der zuerst genannten Druckschrift beschriebene Verfahren geht in seiner optimierten Form davon aus, das Reaktionsrohprodukt mit einer Säurezahl von ca. 40 - bedingt durch Überschüsse an Acrylsäure, p-Toluolsulfonsäure und phenolische Herstellungsinhibitoren, insbesondere Di-tert.-butyl-hydrochinon - bei 80°C mit einem, bezogen auf die Säurezahl, doppelt molaren Überschuß an Calciumhydroxid (wasserfrei) zu versetzen. Dieses Gemisch läßt man dann ca. 1 Stunde reagieren, dann wird das Neutralisationswasser abgezogen und das Reaktionsgemisch durch Filtration von Calciumsalzen der genannten sauren Komponenten befreit.

Die Vorteile dieses Verfahrens liegen in der nahezu völligen Entfernung der sauren Bestandteile, in einer guten Produktfarbe nach der Filtration und der Konzentration aller Nebenbestandteile in dem Filterkuchen.

Die Nachteile dieser Ausgestaltung der Trockenneutralisation liegen in relativ langen Filtrationszeiten und vergleichweise doch berträchtlichen Verlusten des Wertproduktes im Filterkuchen. So können nach dem Verfahren, wie es in der DE-A1 39 39 162 beschrieben ist, im 1 kg-Laborversuch Filtrationszeiten von mehreren Stunden (üblicherweise 2 bis 4 Stunden) und Produktverluste zwischen 10 und 15 Gew.-% auftreten.

Aus DE-A1 38 43 930 war ein Verfahren zur Reinigung von rohen Estern bekannt, bei dem das Neutralisationsmittel entweder als Lösung oder in festem Zustand ohne Zugabe von Wasser zugesetzt wurde.

Aus DE-A1-23 30 435 war ein Verfahren zur Neutralisation roher Ester aus Säuren ohne olefinische Doppelbindungen durch Neutralisation mit wäßrigen Lösungen der Neutralisationsmittel bekannt.

Die Lehre der vorliegenden Erfindung geht von der Aufgabe aus, die hier dargestellten Mängel der Trockenneutralisation substantiell zu mindern, ohne die Vorteile des geschilderten Verfahrenstyps grundsätzlich aufgeben zu müssen. Insbesondere will die erfindungsgemäße Lehre des Herstellungsverfahren der nicht destillierbaren und vergelungsgefährdeten Reaktionsprodukte der geschilderten Art so optimieren, daß bei geringen Filtrationszeiten und geringem Aufwand an die Filter geringere Filterkuchenmengen und konsequenterweise geringe Verluste an Wertprodukt anfallen.

Die erfindungsgemäße Lehre geht von der Feststellung aus, daß in der Neutralisationsstufe und der nachfolgenden Abtrennung des Filterkuchens in der hier geschilderten Art dann substantielle Erleichterungen eingestellt werden können, wenn geringe Wassermengen in diese Neutralisationsstufe eingeführt und dabei eine im nachfolgenden im einzelnen geschilderte Sequenz von Verfahrensschritten eingehalten wird. Als Ergebnis des erfindungsgemäßen Handelns werden insbesondere 2 wichtige Verbesserungen erreicht: Die Zeitdauer der Neutralisation kann substantiell abgekürzt werden, gleichzeitig gelingt es, die Menge des abzutrennenden Feststoffanteils und damit des entstehenden Filterkuchens substantiell zu verringern. Gleichzeitig damit wird der unvermeidbare Produktverlust entsprechend stark herabgesetzt, ohne daß auf unerwünschte Hilfs- bzw. Arbeitsmittel wie Lösungsmittel, Waschverfahren und dergleichen zurückgegriffen werden müßte.

### Gegenstand der Erfindung

Gegenstand der Erfindung ist dementsprechend ein Verfahren zur Reingewinnung von im wesentlichen neutralen, schwerflüchtigen und reaktive olefinische Doppelbindungen aufweisenden organischen Verbindungen aus einem Einsatzgut, das diese Komponenten zusammen mit untergeordneten Mengen saurer Reaktionsbestandteile und/oder entsprechender Hilfsstoffe in Flüssigphase enthält, mittels Neutralisation, Abtrennung der gebildeten Salze und Gewinnung von Reinprodukten, die auch ohne Destillation niedrige Rest-Säurezahlen mit niedrigen Farbzahlen verbinden, unter Einsatz von feinpulvrigen Oxiden, Carbonaten und/oder Hydroxiden der Alkali- und/oder Erdalkalimetalle - im nachfolgenden als "Neutralisationsmittel" bezeichnet - sowie nachfolgende Abtrennung der organischen Flüssigphase von der gebildeten Feststoffphase.

Das erfindungsgemäße Verfahren ist dadurch gekennzeichnet, daß man den Neutralisationsschritt wie folgt ausgestaltet:
- Zugabe des festen Neutralisationsmittels zum heißen Einsatzgut, wobei - bezogen auf die Säurezahl des Einsatzgutes - das Neutralisationsmittel in wenigstens stöchiometrischer Menge zur Anwendung kommt,
- gleichzeitig oder nachfolgend Zugabe einer beschränkten Menge an Wasser zum Reaktionsgemisch,
- Durchführung und wenigstens weitgehender Abschluß der Neutralisation durch Vermischen des Stoffgemisches für den Zeitraum von wenigstens einigen Minuten,
- Abdestillieren eines wenigstens substantiellen Anteiles des Wassers aus dem heißen Reaktionsgut bei vermindertem Druck, sowie schließlich
- Abtrennung der jetzt im Reaktionsgut vorliegenden Feststoffphase auf beliebigem Wege, zweckmäßigerweise durch Filtration.

Die Erfindung betrifft dabei in einer Ausführungsform die Anwendung dieses Verfahrens für die Herstellung technischer Produkte, die beispielsweise als UV-härtbare Lacksysteme praktische Bedeutung haben können. In einer wichtigen weiteren Ausführungsform will die Erfindung insbesondere aber auch den teilweisen oder bevorzugt vollständigen Austausch von Radikalinhibitoren - sogenannte Herstellungsinhibitoren - aus ihren Abmischungen mit radikalisch reaktiven, insbesondere polymerisierbaren und/oder vernetzbaren 1-und/oder mehrfach olefinisch ungesättigten Verbindungen gegen nach Art und Menge frei bestimmbare radikalische Inhibitoren oder Inhibitor-Systeme - die im nachfolgenden auch als Applikationsinhibitoren bezeichnet werden - ermöglichen. Wichtig ist diese Anwendung des erfindungsgemäßen Verfahrens für den Austausch von Herstellungsinhibitoren begrenzter physiologischer Verträglichkeit gegen physiologisch besser verträgliche Applikationsinhibitoren. Wichtige praktische Bedeutung kann dieser Aspekt der Erfindung insbesondere im Zusammenhang mit härtbaren und/oder ausgehärteten Systemen bzw. Formteilen, wie Klebstoffen oder festen operativen Hilfsstoffen, zukommen, die für den unmittelbaren Kontakt mit dem menschlichen und/oder tierischen Körper gedacht sind.

### Einzelheiten zur erfindungsgemäßen Lehre

Ein wesentliches Element des erfindungsgemäßen Verfahrens liegt in der gezielten Zugabe von geringen Mengen an Wasser zum Reaktionsgut zur Erleichterung der Neutralisationsreaktion und der nachfolgenden Filtration. Für die Optimierung des Arbeitsergebnisses ist dabei allerdings auch die Reihenfolge in der Zugabe des festen Neutralisationsmittels und des im erfindungsgemäßen Sinne zugegebenen geringen Wasseranteiles bedeutungsvoll. Die Lehre der Erfindung sieht hier vor, zunächst das feste Neutralisationsmittel in der stöchiometrisch wenigstens etwa benötigten Menge in das zu neutralisierende Reaktionsrohmaterial einzumischen und erst jetzt das Wasser als Reaktionshilfsmittel beizugeben. Vergleichsversuche haben gezeigt, daß durch eine Umkehr der zeitlichen Reihenfolge dieser Verfahrensschritte die Vergelungsgefahr in Abhängigkeit von der Wassermenge steigt bzw. das Wasser seine Eigenschaft als Hilfsstoff nicht entwickeln kann, während bei gleichzeitiger Zugabe von Wasser und Neutralisationsmittel zum Einsatzgut häufig in der nachfolgenden Trennstufe längere Filtrationszeiträume benötigt werden. Gleichwohl umfaßt die Lehre der Erfindung eine solche gleichzeitige Zugabe von Neutralisationsmittel und Wasser, wobei es allerdings bevorzugt sein kann, wenigstens einen Anteil des festen Neutralisationsmittels zunächst zuzugeben.

Die Arbeitsschritte des erfindungsgemäßen Verfahrens können an sich in einem vergleichsweise breiten Bereich von Arbeitstemperaturen durchgeführt werden. In Betracht kommen etwa Temperaturen im Bereich von etwa Raumtemperatur bis 140°C. Die Auswahl der jeweils optimalen Temperaturen wird unter anderem durch die Viskosität der zu behandelnden Flüssigphasen mitbestimmt. So kann es bevorzugt sein, das Einsatzgut während der genannten Arbeitsschritte bei erhöhten Temperaturen, insbesondere bei Temperaturen von wenigstens 50°C zu halten. Andererseits sind zu hohe Arbeitstemperaturen zu vermeiden, wobei hier als Obergrenze insbesondere 100 bis 120°C zu nennen sind. Für das praktische Arbeiten haben sich Temperaturen innerhalb des Bereiches von 70 bis 90°C und hier insbesondere Arbeitstemperaturen im Bereich um etwa 80°C als besonders zweckmäßig erwiesen. Beeinflußt werden kann durch die richtige Temperaturwahl einerseits die Dauer der Reaktionszeit, sowie die Dauer der Filtrationsstufe, andererseits ist hier aber auch die Farbe des Reaktionsgutes zu berücksichtigen, die letztlich angestrebt wird. In einer Reihe von Einsatzgebieten - beispielsweise bei der Herstellung von Klarlacken - wird Wert auf die Hellfarbigkeit des letztlich zum Einsatz kommenden Reaktionsgutes gelegt. Über die Temperatursteuerung des erfindungsgemäßen Verfahrens kann hier insbesondere auf diesen Parameter Einfluß genommen werden.

Eine weitere bevorzugte Maßnahme des erfindungsgemäßen Verfahrens kann insbesondere dann wichtig sein, wenn stark vergelungsgefährdete Reaktionsprodukte der Behandlung unterworfen werden sollen, die mit aerob wirksamen Inhibitoren - beispielsweise solchen vom Phenoltyp - inhibiert und damit nur in Anwesenheit von Sauerstoff wirksam sind. Hier sieht die Lehre der Erfindung vor, daß man das Einsatzgut wenigstens während der Zugabe von Neutralisationsmittel und Wasser und während der nachfolgenden Neutralisation mit Luft begast. So kann es zweckmäßig sein, beispielsweise das Rohprodukt im Reaktor auf etwa 80°C zu erhitzen und dabei Luft einzuleiten. Die nachfolgenden Reaktionsschritte werden ebenfalls unter fortgesetztem Einleiten von Luft durchgeführt. Auf diese Weise kann einer Gefahr des unerwünschten Vergelens auch hochreaktiver Reaktionsrohprodukte wirkungsvoll begegnet werden. Bei der Verwendung von anaerob wirksamen Inhibitoren, zum Beispiel Phenothiazin, muß die Begasung mit Luft unterbleiben.

Das im ersten Verfahrensschritt zugesetzte feste Neutralisationsmittel wird in seiner Menge bevorzugt so beschränkt, daß höchstens etwa das 1,5-fache des stöchiometrisch benötigten Betrages zugesetzt wird. Die geringste Menge des Neutralisationsmittels entspricht dem stöchiometrischen Betrag, wenn die vollständige Neutralisation der zur Salzbildung befähigten sauren Komponenten gefordert wird. In einer besonders wichtigen Ausführungsform der erfindungsgemäßen Lehre wird der Mengenanteil des festen Neutralisationsmittels so bemessen, daß das 1,1 bis 1,3-fache der stöchiometrisch jeweils benötigten Mengen eingesetzt wird. Auf diese Weise wird die Menge des letztlich anfallenden Filterkuchens auf das notwendige Maß eingeschränkt, gleichzeitig - unter Mitwirken des erfindungsgemäß eingesetzten Wasserbetrages - aber sichergestellt, daß eine vollständige Neutralisation eintritt.

Gleichzeitig mit oder bevorzugt nach der Zugabe des wenigstens weitgehend trockenen Neutralisationsmittels wird dem heißen Reaktionsansatz das Wasser in Mengen von 0,5 bis 15 Gew.-% und vorzugsweise in Mengen von 2 bis 10 Gew.-% zugegeben, wobei hier sich die Zahlenwerte in Gew.-% auf das Einsatzgut beziehen. Besonders geeignet kann die Zugabe von 3 bis 7 Gew.-% Wasser zum Einsatzgut sein. In der Praxis wird man häufig mit 5 Gew.-% Wasser arbeiten. Größere Wassermengen führen zu einem Anstieg der Zeitspanne, die für das nachträgliche Abdestillieren des Wassers benötigt wird. Damit können Produktverluste verbunden sein. Geringere Wassermengen können insbesondere die Filtrationszeit negativ beeinflussen.

Der zum Abschluß der Neutralisationsreaktion unter den erfindungsgemäßen Arbeitsbedingungen benötigte Zeitraum beträgt üblicherweise nicht mehr als höchstens etwa 30 Minuten und liegt im allgemeinen im Bereich von 10 bis 20 Minuten. Während dieser Reaktionsphase ist auf eine hinreichende Durchmischung des mehrphasigen Reaktionsgemisches zu achten. Unter Berücksichtigung aller dieser Variablen stellt sich die erfindungsgemäße Modifikation der Neutralisationsstufe beispielsweise wie folgt dar:

Das Reaktionsrohprodukt im Reaktor wird auf 80°C erhitzt und dabei Luft eingeleitet. Zu dem Rohprodukt wird (bezogen auf die Ausgangssäurezahl) die etwa 1,2-fache Menge eines Hydroxids von Alkali- oder Erdalkalimetallen in fester Form zugegeben. Im direkten Anschluß werden etwa 5 Gew.-% Wasser zum Gemisch zugegeben, dann wird bei 80°C unter Rühren für den Zeitraum von etwa 15 Minuten neutralisiert. Nach dieser Neutralisationsstufe wird das Wasser - sowohl das entstandene Neutralisationswasser als auch wenigstens anteilsweise das erfindungsgemäß bewußt zugesetzte Wasser - bei 80°C im Laufe von 15 bis 30 Minuten abdestilliert.

Es liegt jetzt eine Aufschlämmung eines festen Salzes im neutralisierten flüssigen Reaktionsgut vor. Dieses Stoffgemisch wird der Phasentrennung unterworfen, wobei zweckmäßigerweise der Feststoffanteil über Filter abgetrennt wird. Geeignet sind hier insbesondere Druckfilter, die beispielsweise bei einem Arbeitsdruck von einigen bar Überdruck zum Einsatz kommen können.

Gegenüber den Trockenneutralisationsverfahren der eingangs geschilderten Literatur können im erfindungsgemäßen Neutralisationsverfahren substantielle Verbesserungen sowohl bezüglich der erforderlichen Filtrationszeit als auch bezüglich der Produktverluste erzielt werden. So kann beispielsweise in Vergleichsansätzen die Filtrationsdauer von ca. 1 Stunde auf den Zeitraum von 2 Minuten herabgesetzt werden. Die über den abgetrennten Filterkuchen auftretenden Produktverluste liegen deutlich unter 10 bis 15 Gew.-%, beispielsweise im Bereich von ca. 3 Gew.-% - jeweils bezogen auf Reaktionsrohprodukt.

Besondere Bedeutung kommt der Verwendung von feinteiligen, insbesondere feinpulvrigen 0xiden und/oder Hydroxiden von Calcium und/oder Magnesium zur Durchführung des Verfahrens zu. Hierbei gilt, daß von diesen beiden Erdalkalimetallen den entsprechenden Verbindungen des Calciums wiederum besondere Bedeutung zukommt. In einer bevorzugten Ausführungsform der Erfindung werden feinteilige Feststoffe in die Neutralisationsstufe eingeführt, die wenigstens anteilsweise Calciumverbindungen der genannten Art enthalten. In einer besonders wichtigen Ausführungsform wird Calciumhydroxid Ca(OH)₂ und/oder gebrannter Kalk Ca0 eingesetzt. Insbesondere feinteiliges Calciumhydroxid ermöglicht häufig eine optimal ausgleichende Einstellung der sich eigentlich gegensätzlich auswirkenden Verfahrensparameter bezüglich Restsäurezahl, Farbzahl, thermischer Belastbarkeit, Verfahrensdauer, Menge des einzusetzenden trockenen Neutralisationsmittels und dergleichen.

Zum Abdestillieren des Wassers nach der Neutralisationsreaktion wird zweckmäßigerweise bei hinreichend verringerten Drucken im angegebenen Temperaturbereich oberhalb 50°C gearbeitet. Geeignet sind Druckbereiche von 1 bis 150 mbar, wobei das Arbeiten im Bereich von 20 bis 150 mbar und insbesondere im Bereich von 20 bis 100 mbar besonders bevorzugt sein kann. Es gelingt auf diese Weise, Restwassergehalte im Fertigprodukt auf Werte unter 0,1 Gew.-% abzusenken.

Wie bereits angegeben, ist die erfindungsgemäße Modifikation des Neutralisationsschrittes nicht nur für die Herstellung von technischen Großprodukten wichtig, wie sie beispielsweise in der DE-A1 39 39 163 geschildert sind, besondere Bedeutung kann die erfindungsgemäße Modifikation auch für den Inhibitoraustausch im Sinne der Lehre der DE-A1 39 39 162 besitzen. Möglich ist das immer dann, wenn als sogenannte Herstellungsinhibitoren Verbindungen eingesetzt werden, die unter den Bedingungen der Neutralisationsreaktion zur Salzbildung und damit zur Anbindung an den abzutrennenden Filterkuchen geeignet sind. Im einzelnen kann auf die genannten Druckschriften verwiesen werden. Hier sei nur auszugsweise wiederholt: In der hier betroffenen Ausführungsform gelingt mittels der Neutralisationsbehandlung und der hier gegebenen Möglichkeit der einfachen Phasentrennung die Abreicherung oder auch die vollständige Entfernung des Herstellungsinhibitors aus dem Einsatzgemisch. Dadurch wird die freie Wahl des Applikationsinhibitors nach Art und/oder Menge möglich.

Phenolverbindungen mit zur Salzbildung befähigten freien Hydroxylgruppen sind eine in der Polymerisationstechnik bekannte Klasse von Verbindungen mit ausgeprägter aerober Inhibitorwirksamkeit gegen radikalische Reaktionsauslösung in olefinisch ungesättigten Systemen. Besonders wirksame Vertreter finden sich in der Klasse der Hydrochinonverbindungen, insbesondere der ringsubstituierten Hydrochinonverbindungen. Alpha-substituierte Hydrochinone und hier wiederum Dialkyl-substituierte Verbindungen dieser Art sind besonders reaktiv und eignen sich dementsprechend zum Einsatz als Herstellungsinhibitor besonders. Diese Inhibitorklasse zeichnet sich durch hohe Hemmwirkung gegen unerwünschte radikalische Polymerisation aus. Ein besonders stark hemmwirksamer Vertreter ist beispielsweise das 2,5-Di-tert.-Butyl-hydrochinon, das in praktisch frei wählbaren Mengen in der Stufe der Herstellung der Reaktivsysteme zur Stabilisierung eingesetzt werden kann. Durch die nachgeschaltete Abtrennung im Sinne des erfindungsgemäßen Handelns solcher salzbildenden Inhibitoren über ihre wenigstens anteilsweise Anbindung an ein festes Fällungsmittel und nachfolgende Phasentrennung wird es möglich, den Herstellungsinhibitor nach Art und/oder Menge gegen frei gewählte Applikationsinhibitoren auszutauschen.

Besondere Bedeutung kann dieser Austausch immer dann bekommen, wenn die Reaktivsysteme in der Anwendung einer Überprüfung ihrer physiologischen Verträglichkeit bedürfen, wie es beispielsweise im Rahmen der Klebstofftechnologie im lebenden Organismus der Fall ist. Hier werden Klebstoffe bzw. Klebstoffsysteme gesucht, die physiologisch hochvertägliche Applikationsinhibitoren enthalten. Ein besonders geeineter Inhibitor ist das Vitamin E, dessen Verwendung für Zwecke der hier angeschnittenen Art im einzelnen beschrieben wird in der DE-A1 39 39 161.

### Beispiele

### Beispiel 1

Ein Reaktionsrohprodukt (1000 g), hergestellt durch lösungsmittelfreie Veresterung von Trimethylolpropan+3EO gemäß der Lehre der DE-A1 38 43 854 mit 20 Mol-% Überschuß an Acrylsäure unter Verwendung von 3,5 Gew.-% p-Toluolsulfonsäure als Katalysator und 2000 ppm 2,5-Di-tert.-butylhydrochinon als Inhibitor, mit einer Säurezahl von 30 wird auf 80°C erhitzt. Es wird die 1,5-fache stöchiometrische Menge an festem pulvrigem Calciumhydroxid in einer Portion zugegeben. Danach erfolgt die Zugabe von 10 Gew.-% Wasser, bezogen auf die Gesamtmenge. Es wird 30 Minuten gerührt. Anschließend wird das Wasser bei 50 mbar innerhalb von 30 Minuten unter Rühren entfernt (Propellerrührer mit 400 UpM), wobei das Produkt mit 40 l/h Luft durchströmt wird. Die jetzt vorliegende Feststoffphase wird über einen 2 l-Druckfilter (0,01 m² Filterfläche) bei 3 bar abgetrennt. Es wird ein Produkt mit den folgenden Kennwerten erhalten: Säurezahl 0,05, Farbzahl (Gardner) <1, Wassergehalt 0,4 Gew.-%. Die Filtrationszeit beträgt ca. 20 min, der Produktverlust macht etwa ca. 6 Gew.-% aus.

### Beispiel 2

1000 g eines Reaktionsrohproduktes, hergestellt gemäß den Angaben des Beispiels 1, jedoch unter Einsatz von Polyethylenglykol als mehrfunktionellem Alkohol, mit einer Säurezahl von 30 werden auf 80°C erhitzt. Es wird die 1,3-fache stöchiometrische Menge an festem pulvrigem Calciumhydroxid und die 0,5-fache stöchiometrische Menge an festem pulvrigem Magnesiumhydroxid in einer Portion zugegeben. Danach erfolgt die Zugabe von 3 Gew.-% Wasser - bezogen auf die Gesamtmenge. Es wird 30 Minuten gerührt. Anschließend wird das Wasser bei 50 mbar innerhalb von 30 Minuten unter Rühren (Propellerrührer 400 UpM) entfernt, wobei das Produkt mit 40 l/h Luft durchströmt wird. Die jetzt vorliegende Feststoffphase wird über einen 2 l-Druckfilter (0,01 m² Filterfläche) bei 3 bar abgetrennt. Es wird ein Produkt mit den folgenden Kenngrößen erhalten: Säurezahl 0,2, Farbzahl (Gardner) <1, Wassergehalt 0,15 Gew.-%. Die Filtrationszeit beträgt ca. 2 min, der Produktverlust macht ca. 3 Gew.-% aus.

### Beispiel 3

Das Reaktionsrohprodukt des Beispiels 1 mit seiner Säurezahl von 30 wird jetzt in einer Menge von 100 kg eingesetzt und auf 80°C erhitzt. Anschließend wird die 1,2-fache stöchiometrische Menge an festem pulvrigem Calciumhydroxid in einer Portion zugegeben. Danach erfolgt die Zugabe von 5 Gew.-% Wasser, bezogen auf die Gesamtmenge. Es wird für 30 Minuten gerührt. Anschließend wird das Wasser bei 50 mbar innerhalb von 30 Minuten unter Rühren (4-stufiger MIG-Rührer) entfernt, wobei die flüssige Produktphase mit 400 l/h Luft durchströmt wird. Die gebildete Feststoffphase wird über einen 160 l-Druckfilter (0,5 m² Filterfläche) bei 2 bar abgetrennt. Es wird ein Produkt mit den folgenden Kennzahlen erhalten: Säurezahl 0,1, Farbzahl (Gardner) <1, Wassergehalt 0,3 Gew.-%. Die Filtrationszeit beträgt ca. 11 min, der Produktverlust beträgt ca. 3,5 Gew.-%.

### Beispiel 4

1000 g eines Reaktionsrohproduktes gemäß den Angaben des Beispiels 1, jedoch unter Verwendung von Neopentylglykol+2PO als polyfunktionelle Alkoholkomponente mit einer Säurezahl von 30 werden auf 80°C erhitzt. Anschließend wird die 0,9-fache stöchiometrische Menge an festem pulvrigem Calciumhydroxid und die 0,5-fache stöchiometrische Menge an festem Magnesiumhydroxid in einer Portion zugegeben. Danach erfolgt die Zugabe von 3 Gew.-% Wasser, bezogen auf die Gesamtmenge und 30 minütiges Rühren. Anschließend wird das Wasser bei 50 mbar innerhalb von 30 Minuten unter Rühren (Propellerrührer 400 UpM) entfernt, wobei das Flüssigprodukt mit 40 l/h Luft durchströmt wird. Die gebildete Feststoffphase wird über einen 2 l-Druckfilter (0,01 m² Filterfläche) bei 3 bar abgetrennt. Das erhaltene Produkt weist die folgenden Parameter auf: Säurezahl 0,9, Farbzahl (Gardner) 1, Wassergehalt 0,5 Gew.-%. Die Filtrationszeit beträgt ca. 1 min, der Produktverlust macht ca. 3 Gew.-% aus.

### Beispiel 5

Das Reaktionsrohprodukt des Beispiels 1 wird in einer Menge von 1000 g auf 90°C erhitzt. Es wird die 1,2-fache stöchiometrische Menge an festem pulvrigem Calciumhydroxid zusammen mit 5 Gew.-% Wasser - bezogen auf die Gesamtmenge - als Aufschlämmung in einer Portion zugegeben. Danach wird 30 Minuten gerührt. Anschließend wird das Wasser bei 50 mbar innerhalb von 30 Minuten unter Rühren (Propellerrührer 400 UpM) entfernt, wobei das Produkt mit 40 l/h Luft durchströmt wird. Die gebildete Feststoffphase wird über einen 2 l-Druckfilter (0,01 m² Filterfläche) bei 3 bar abgetrennt. Es wird ein Produkt mit den folgenden Kennzahlen erhalten: Säurezahl 0,3, Farbzahl (Gardner) <1, Wassergehalt 0,5 Gew.-%. Die Filtrationszeit beträgt ca. 2 min, der Produktverlust macht ca. 3 Gew.-% aus.

### Beispiel 6

1000 g eines Reaktionsrohproduktes, hergestellt analog Beispiel 1, jedoch unter Verwendung von Bisphenol A als polyfunktionelle Alkoholkomponente und einer Säurezahl von 25, wird auf 80°C erhitzt. Es wird die 1,5-fache stöchiometrische Menge an festem pulvrigem Calciumhvdroxid in einer Portion zugegeben. Danach erfolgt die Zugabe von 2 Gew.-% Wasser - bezogen auf die Gesamtmenge -, dann wird 30 Minuten gerührt. Anschließend wird das Wasser bei 50 mbar innerhalb von 30 Minuten unter Rühren (Propellerrührer 400 UpM) entfernt, wobei das Produkt mit 40 l/h Luft durchströmt wird. Die gebildete Feststoffphase wird über einen 2 l-Druckfilter (0,01 m² Filterfläche) bei 3 bar abgetrennt. Es wird ein Produkt mit den folgenden Parametern erhalten: Säurezahl 0,2, Farbzahl (Gardner) 3 bis 4, Wassergehalt 0,5 Gew.-%. Die Filtrationszeit beträgt ca. 5 min, der Produktverlust beträgt ca. 4 Gew.-%.

## Patentansprüche

1. Verfahren zur Reingewinnung von im wesentlichen neutralen, schwerflüchtigen und reaktive olefinische Doppelbindungen aufweisenden organischen Verbindungen aus einem Einsatzgut, das diese Komponenten zusammen mit untergeordneten Mengen saurer Reaktionsbestandteile und/oder entsprechender Hilfsstoffe in Flüssigphase enthält, mittels Neutralisation, Abtrennung der gebildeten Salze und Gewinnung von Reinprodukten, die auch ohne Destillation niedrige Rest-Säurezahlen mit niedrigen Farbzahlen verbinden, unter Einsatz von festen feinpulvrigen Oxiden, Carbonaten und/oder Hydroxiden der Alkali- und/oder Erdalkalimetalle (Neutralisationsmittel) und nachfolgender Abtrennung der organischen Flüssigphase von der gebildeten Feststoffphase, dadurch gekennzeichnet, daß man den Neutralisationsschritt wie folgt ausgestaltet:
- Zugabe des festen Neutralisationsmittels in - bezogen auf die Säurezahl des Einsatzgutes - wenigstens etwa stöchiometrischer Menge zum heißen Einsatzgut,
- gleichzeitig oder nachfolgend Zugabe einer Wassermenge von 0,5 bis 15 Gew.-% bezogen auf das Einsatzgut,
- Neutralisation durch Vermischen für den Zeitraum von wenigstens einigen Minuten,
- Abdestillieren des Wassers aus dem heißen Reaktionsgut bei vermindertem Druck und
- Abtrennung der jetzt vorliegenden Feststoffphase vom flüssigen Gut.

2. Verfahren nach Anspruch 1, dadurch gekennzeichnet, daß man das Einsatzgut während der genanten Arbeitsschritte im Bereich von Raumtemperatur bis 140°C hält.

3. Verfahren nach Ansprüchen 1 und 2, dadurch gekennzeichnet, daß man beim Arbeiten mit aerob wirksamen Inhibitoren das Einsatzgut wenigstens während der Zugabe von Neutralisationsmittel und Wasser, sowie der anschließenden Neutralisatiationsstufe mit Luft begast.

4. Verfahren nach Ansprüchen 1 bis 3, dadurch gekennzeichnet, daß man die Menge des Neutralisationsmittels - bezogen auf die Säurezahl des Einsatzgutes - auf höchstens das 1,5-fache des stöchiometrisch benötigten Betrages einschränkt.

5. Verfahren nach Ansprüchen 1 bis 4, dadurch gekennzeichnet, daß nach oder gleichzeitig mit der Zugabe des wenigstens weitgehend trockenen Neutralisationsmittels Wasser in Mengen von 2 bis 10 Gew.-% - bezogen aufEinsatzgut - zugegeben wird.

6. Verfahren nach Ansprüchen 1 bis 5, dadurch gekennzeichnet, daß man die Abtrennung des Feststoffanteils durch Druckfiltration vornimmt.

7. Verfahren nach Ansprüchen 1 bis 6, dadurch gekennzeichnet, daß man die Neutralisation mit den Oxiden und/oder Hydroxiden von Calcium und/oder Magnesium durchführt, wobei die wenigstens anteilsweise Mitverwendung entsprechender Calciumverbindungen bevorzugt ist.

8. Verfahren nach Ansprüchen 1 bis 7, dadurch gekennzeichnet, daß man das Abdestillieren des Wassers aus dem heißen Rekationsgut bei Enddrucken im Bereich von 1 bis 150 mbar durchführt.

9. Verfahren nach Ansprüchen 1 bis 8, dadurch gekennzeichnet, daß man mit wenigstens weitgehend wasserfreien polymerisations- bzw. vergelungs-gefährdeten olefinisch ungesättigten Einsatzmaterialien arbeitet, die als saure Verunreinigungen Restsäureanteile aus der Kondensation, saure Katalysatoren, sauer reagierende Inhibitoren und dergleichen enthalten.

10. Verfahren nach Ansprüchen 1 bis 9, dadurch gekennzeichnet, daß als Einsatzmaterialien schwerflüchtige Ester aus polyfunktionellen Alkoholen und olefinisch ungesättigten Carbonsäuren verwendet werden.

11. Anwendung des Verfahrens nach Ansprüchen 1 bis 10 zum Inhibitoraustausch, bei dem ein zur Salzbildung mit Basen befähigter Radikalinhibitor (Herstellungsinhibitor) gegen nach Art und Menge frei bestimmbare radikalische Inhibitoren oder Inhibitor-Systeme (Applikationsinhibitor) ausgetauscht wird.

12. Ausführungsform nach Anspruch 11, dadurch gekennzeichnet, daß man reaktive Ausgangsmaterialien dem Inhibitoraustausch unterwirft, die Phenol- und/oder Hydrochinonverbindungen, insbesondere alpha-substituierte Hydrochinone als Herstellungsinhibitor enthalten.

13. Ausführungsform nach Ansprüchen 11 und 12, dadurch gekennzeichnet, daß man den Inhibitoraustausch an Stoffgemischen durchführt, die Dialkyl-substituierte Hydrochinone und insbesondere 2,5-Di-tert.-butyl-hydrochinon als Herstellungsinhibitor enthalten.

14. Ausführungsform nach Ansprüchen 11 bis 13, dadurch gekennzeichnet, daß man Herstellungsinhibitoren begrenzter physiologischer Verträglichkeit gegen physiologisch besser verträgliche Applikationsinhibitoren austauscht, wobei als physiologisch verträglicher Applikationsinhibitor insbesondere Vitamin E gewählt wird.

## Claims

1. A process for recovering substantially neutral, low-volatility organic compounds containing reactive olefinic double bonds in pure form from a starting material which contains these components together with small quantities of acidic reaction components and/or corresponding auxiliaries in the liquid phase by neutralization, removal of the salts formed and recovery of pure products which - even without distillation - combine low residual acid values with low colour values using solid, finely powdered oxides, carbonates and/or hydroxides of the alkali and/or alkaline earth metals (neutralizing agents) and subsequent removal of the organic liquid phase from the solid phase formed, characterized in that the neutralization step is carried out as follows:
- addition of the solid neutralizing agent to the hot starting material in an at least substantially stoichiometric quantity, based on the acid value of the starting material,
- simultaneous or subsequent addition of water in a quantity of 0.5 to 15% by weight, based on the starting material,
- neutralization by mixing for at least a few minutes,
- removal of the water from the hot reaction product by distillation under reduced pressure and
- removal of the solid phase now present from the liquid.

2. A process as claimed in claim 1, characterized in that the starting material is kept at a temperature from room temperature to 140°C during the steps mentioned above.

3. A process as claimed in claims 1 and 2, characterized in that, where aerobic inhibitors are used, the starting material is aerated at least during the addition of the neutralizing agent and water and the subsequent neutralization step.

4. A process as claimed in claims 1 to 3, characterized in that the quantity of neutralizing agent, based on the acid value of the starting material, is limited to at most 1.5 times the stoichiometrically necessary amount.

5. A process as claimed in claims 1 to 4, characterized in that water is added in quantities of 2 to 10% by weight, based on the starting material, after or at the same time as the addition of the at least substantially dry neutralizing agent.

6. A process as claimed in claims to 5, characterized in that the solid component is removed by pressure filtration.

7. A process as claimed in claims 1 to 6, characterized in that neutralization is carried out with the oxides and/or hydroxides of calcium and/or magnesium, the at least partial use of corresponding calcium compounds being preferred.

8. A process as claimed in claims 1 to 7, characterized in that the removal of the water from the hot reaction product by distillation is carried out at end pressures of 1 to 150 mbar.

9. A process as claimed in claims 1 to 8, characterized in that at least substantially water-free, olefinically unsaturated starting materials susceptible to polymerization or gelation which contain residual acid from the condensation reaction, acidic catalysts, inhibitors with an acidic reaction and the like as acidic impurities are used.

10. A process as claimed in claims 1 to 9, characterized in that low-volatility esters of polyhydric alcohols and olefinically unsaturated carboxylic acids are used as the starting materials.

11. The use of the process claimed in claims 1 to 10 for inhibitor exchange, in which a radical inhibitor (production inhibitor) capable of forming salts with bases is exchanged for radical inhibitors or inhibitor systems (application inhibitor) which may be freely selected in regard to type and quantity.

12. The use claimed in claim 11, characterized in that reactive starting materials containing phenolic and/or hydroquinone compounds, more especially α-substituted hydroquinones, as production inhibitors are subjected to the inhibitor exchange.

13. The use claimed in claims 11 and 12, characterized in that the inhibitor exchange is carried out with mixtures containing dialkyl-substituted hydroquinones and, in particular, 2,5-di-tert.butyl hydroquinone as the production inhibitor.

14. The use claimed in claims 11 to 13, characterized in that production inhibitors with limited physiological compatibility are exchanged for application inhibitors with better physiological compatibility, vitamin E in particular being used as the physiologically compatible application inhibitor.

## Revendications

1. Procédé de récupération pure de composés organiques essentiellement neutres, peu volatiles et présentant des doubles liaisons oléfiniques réactives, à partir d'un produit de départ, qui contient ces composants en phase liquide, conjointement avec des quantités secondaires de constituants réactionnels acides et/ou d'adjuvants correspondants, par neutralisation, séparation des sels formés et récupération des produits purs, qui allient même sans distillation de faibles indices d'acidité résiduels à de faibles indices de coloration, moyennant l'utilisation d'oxydes, de carbonates et/ou d'hydroxydes de métaux alcalins et/ou alcalino-terreux en poudre fine (agents neutralisants), et séparation postérieure de la phase liquide organique de la phase solide formée, caractérisé en ce que l'on agence l'étape de neutralisation comme suit:
- adjonction de l'agent neutralisant solide au produit de départ chaud, en quantité au moins stoechiométrique par rapport à l'indice d'acidité du produit de départ.
- adjonction simultanée ou postérieure d'une quantité de 0,5 à 15 % en poids d'eau par rapport au produit de départ.
- neutralisation par mélangeage pendant une période d'au moins quelques minutes,
- élimination par distillation de l'eau du produit réactionnel chaud, sous pression réduite et
- séparation de la phase solide maintenant présente du produit liquide.

2. Procédé selon la revendication 1, caractérisé en ce que l'on maintient le produit de départ pendant les phases de travail citées, dans l'intervalle s'étendant de la température ambiante jusqu'à 140 °C.

3. Procédé selon les revendications 1 et 2, caractérisé en ce qu'en cas de travail avec des inhibiteurs à activités aérobie, on gaze le produit de départ à l'air, au moins pendant l'adjonction de l'agent neutralisant et de l'eau, ainsi qu'au cours de l'étape de neutralisation postérieure.

4. Procédé selon les revendications 1 à 3, caractérisé en ce que l'on limite la quantité de l'agent neutralisant, par rapport à l'indice d'acidité du produit de départ, au maximum à 1,5 fois la quantité stoechiométriquement nécessaire.

5. Procédé selon les revendications 1 à 4, caractérisé en ce que l'on ajoute de l'eau en quantités de 2 à 10 % en poids - par rapport au produit de départ - après ou en même temps que l'adjonction de l'agent neutralisant au moins largement sec.

6. Procédé selon les revendications 1 à 5, caractérisé en ce que l'on procède à la séparation de la fraction de matière solide par filtration sous pression.

7. Procédé selon les revendications 1 à 6, caractérisé en ce que l'on réalise la neutralisation avec des oxydes et/ou des hydroxydes de calcium et/ou de magnésium, la co-utilisation au moins partielle de composés de calcium correspondants étant préférée.

8. Procédé selon les revendications 1 à 7, caractérisé en ce que l'on réalise l'élimination par distillation de l'eau du produit réactionnel chaud, sous des pressions finales situées dans l'intervalle de 1 à 150 mbars.

9. Procédé selon les revendications 1 à 8, caractérisé en ce que l'on travaille avec des matières de départ menacées par la polymérisation ou la gélification, au moins largement anhydres, qui contiennent comme impuretés acides, des fractions d'acides résiduels provenant de la condensation, des catalyseurs acides, des inhibiteurs à réaction acide, etc.

10. Procédé selon les revendications 1 à 9, caractérisé en ce que l'on utilise comme matières de départ, des esters peu volatiles d'alcools polyfonctionnels et d'acides carboxyliques à insaturation oléfinique.

11. Utilisation du procédé selon les revendications 1 à 10 pour l'échange d'inhibiteurs, par lequel un inhibiteur de radicaux (inhibiteur de fabrications) apte à la salification avec des bases est échangé contre des inhibiteurs ou des systèmes d'inhibiteurs radicalaires (inhibiteurs d'application) dont la nature et la quantité sont librement déterminables.

12. Forme de réalisation selon la revendication 11, caractérisée en ce que l'on soumet à l'échange d'inhibiteurs des matières de départ réactives, qui renferment comme inhibiteurs de fabrication, des composés de phénol et/ou d'hydroquinone, en particulier des hydroquinones à substitution alpha.

13. Forme de réalisation selon les revendications 11 et 12, caractérisée en ce que l'on opère l'échange d'inhibiteurs sur des mélanges de substances, qui renferment comme inhibiteurs de fabrication, des hydroquinones à substitution dialkyle et, en particulier de la 2,5-di-tert.-butyl-hydroquinone.

14. Forme de réalisation selon les revendications 11 à 13, caractérisée en ce que l'on échange des inhibiteurs de fabrication à tolérance physiologique limitée contre des inhibiteurs d'application à meilleure tolérance physiologique, la vitamine E étant en particulier sélectionnée comme inhibiteur d'application physiologiquement tolérable.
